# EUROPEAN PATENT SPECIFICATION

(11) **EP 3 711 741 B1**
(45) Date of publication and mention of the grant of the patent: **30.04.2025**
(21) Application number: 18879813.6
(22) Date of filing: 20.02.2018
(51) Int. Cl.: A61K 6/00, A61L 27/38, A61L 27/54, A61L 27/22, A61L 27/36

(54) **METHOD FOR MANUFACTURING COMPLEX FOR BONE GRAFTING AND COMPLEX FOR BONE GRAFTING MANUFACTURED THEREBY**
VERFAHREN ZUR HERSTELLUNG EINES KOMPLEXES FÜR KNOCHENTRANSPLANTAT UND DAMIT HERGESTELLTER KOMPLEX FÜR KNOCHENTRANSPLANTAT
PROCÉDÉ DE FABRICATION DE COMPLEXE POUR GREFFE OSSEUSE ET COMPLEXE POUR GREFFE OSSEUSE FABRIQUÉ SELON LE PROCÉDÉ

(30) Priority: 14.11.2017 KR 20170151599
(43) Date of publication of application: 23.09.2020
(73) Proprietor: Korea Tooth Bank Co., Ltd., Seoul 03011 (KR)
(72) Inventor: UM, In Woong, Seoul 04389 (KR)
(74) Representative: van Dam, Vincent
(86) International application number: PCT/KR2018/002067
(87) International publication number: WO 2019/098459

(56) References cited:
- EP-A1- 1 994 910
- KR-A- 20020 075 605
- KR-A- 20100 040 427
- KR-A- 20140 018 455
- KR-B1- 101 488 716
- US-A1- 2010 086 618
- US-A1- 2014 335 146
- GOMES, M. F.: "Histologic Evaluation of the Osteoinductive Property of Autogenous Demineralized Dentin Matrix on Surgical Bone Defects in Rabbit Skulls Using Human Amniotic Membrane for Guided Bone Regeneration", THE INTERNATIONAL JOURNAL OF ORAL & MAXILLOFACIAL IMPLANTS, vol. 16, no. 4, July 2001 (2001-07-01) - 4 November 2001 (2001-11-04), pages 563 - 571, XP055614352

## Description

### [FIELD OF THE INVENTION]

The present disclosure relates to a method for manufacturing a bone graft composite and a bone graft composite manufactured by the same. More particularly, the present disclosure relates to a method for manufacturing a bone graft composite, which includes forming dentin collagen obtained from human teeth and a growth factor having bone-growth inducing function into a composite, thereby improving alveolar bone regeneration rate, as well as a bone graft composite manufactured by the same.

### [BACKGROUND OF THE INVENTION]

In the medical field in recent years, regeneration of bone tissue defects due to physical, chemical and burn related bone tissue injuries or disease such as inflammation or tumors is significantly regarded. Osteomyelitis due to external force or secondary infection easily occurs at a bone tissue defect site. In addition, when skin, muscle, bone and nerve tissues, etc. are damaged in multiple ways, it is difficult to regenerate the tissue into normal tissue or a normal organ in each layer of the tissue and, instead, the tissue is replaced with scar tissue, resulting in restriction in mobility and functionality.

Conventionally, in order to solve this problem, by implanting and filling bone tissues in another part of a patient body into the damaged part, however, side effects such as bone defects in other normal parts, restriction in mobility and functionality, etc. were caused.

Further, autologous bone and other bone graft materials considerably absorb bone during a healing period and have deteriorated ability to maintain volume, thus requiring a dense bone graft material. In this regard, it is difficult to penetrate pores in the above materials.

Further, a shielding membrane is used to prevent growth of other tissues before healing the defective bone tissue or invasion of external bacteria into the tissue, or a porous scaffold is used to provide a space in which cells of the damaged bone tissue at the defect site can grow. Further, there is an increasing tendency to use a growth factor that leads stem cells required for healing or regeneration to move toward the defect site through chemotaxis, or to manipulate or cultivate stem cells and then transplant the same at the defect site so as to achieve cell growth.

In connection therewith, International Patent Laid-Open Publication No. WO1993/000050 disclosed a pharmaceutical formulation based on bone morphogenetic protein (BMP) that comprises a polymeric matrix component selected from a group consisting of lactic acid, glycolic acid and a mixture thereof, as well as a protein-sequestering component for bone formation, and has described a technology for growing mesenchymal stem cells into bone tissue by rhBMP-2.

In recent years, the US FDA permits license of products that use rhBMP-2 immersed in a carrier using collagenous fibers of bovine ligament and tendon. However, there are disadvantages in that: (1) carried BMP does not stay in place but flows into the body in a short time, that is, BMP has inferior functions as a carrier; (2) in terms of physical properties, BMP is not rigid as a graft material, that is, the BMP has a loose or spongy fibrous texture; (3) the whole fiber shrinks during bone induction; and (4) the BMP should be added to a fibroid material immediately before surgery.

On this account, bone tissue grafting technologies using human body-derived tooth were proposed and, in this regard, Korean Patent No. 10-1488716 disclosed a kit for regeneration of alveolar bone that uses a growth factor or constructs a growth factor (rhBMP-2) along with a tooth implant material to induce regeneration of alveolar bone.

However, the kit for regenerating alveolar bone uses a tooth implant material after partial demineralization, hence leaving behind other minerals besides an element required for bone formation (collagen).

In addition, the alveolar bone regeneration kit forms a composite by hydrating a tooth implant material in an aqueous solution of growth factor (rhBMP-2) and then coating the surface of tooth powder with the growth factor (rhBMP-2), hence entailing a limitation such as deterioration in ability of transferring the growth factor to a defect site.

KR 2014 0018455 A discloses bone grafting material using autogenous teeth and a manufacturing method thereof.

EP 1 994 910 A1 discloses a milled product of extracted tooth usable in highly advanced medical treatment, decalcified powder originating in extracted tooth, a method of preparing composite of decalcified powder with apatite and milling machine.

### [BRIEF SUMMARY OF THE INVENTION]

### [OBJECTIVE OF THE INVENTION]

The present disclosure has been proposed to overcome the aforementioned problems, and an object of the present disclosure is to provide a bone graft composite with enhanced alveolar bone regeneration by completely demineralizing teeth to obtain high purity dentin collagen and improving an ability to transfer a growth factor to a defect site

### [DETAILED DESCRIPTION OF THE INVENTION]

In order to solve the above problems, according to an aspect of the present disclosure, there is provided a method for manufacturing a bone graft composite according to claim 1. According to another aspect of the present disclosure, as a solution to overcome the above problem, there is provided a bone graft composite manufactured by the above method.

### [ADVANTAGEOUS EFFECTS]

With the method for manufacturing a bone graft composite and the bone graft composite manufactured thereby according to the present disclosure, the following effects can be accomplished.

First, in a tooth administration group as well as an administration group of a conventional synthetic bone that never regenerates the alveolar bone, bone inducing ability can be maximized to greatly increase an alveolar bone regeneration rate, and perfection and integration of regenerated bone tissues can also be remarkably improved. Further, complete alveolar bone regeneration is possible so as to decrease patient discomfort and, if shape retention is important in a wide range of bone tissue defect sites, side effects due to deformation or contraction of the bone tissue may be reduced.

Second, high purity dentin collagen obtained is synthesized by complete demineralization of a tooth so that undesired reactions induced by other factors during synthesis or transplantation of a bone graft composite, in particular, immune rejection, can be prevented.

Third, unlike the conventional method of coating the surface of tooth powder with a growth factor, a layer filling method of filling a tissue layer of the tooth powder with a growth factor is adopted, thereby improving an ability to transfer a growth factor to a defect site.

Fourth, the bone graft composite consists of the same component called dentin collagen, maintains the original shape even in the body, and thus can have improved bone tissue inducing effects as compared to existing bone graft materials.

### [BRIEF DESCRIPTION OF THE DRAWINGS]

FIG. 1 is a flowchart illustrating a method for manufacturing a bone graft composite according to an embodiment of the present disclosure.
FIG. 2 is a flowchart illustrating detailed steps of a process S400 of synthesizing the composite shown in FIG. 1.
FIG. 3 is a photograph of a tooth implant material in powder form which is used for bone grafting in the conventional method.
FIG. 4 is a photograph of the tooth implant material in powder form used for bone grafting in the conventional method, wherein the tooth implant material was stained with hematoxylin and eosin and then was photographed by a microscope.
FIG. 5 is a photograph taken by an electron microscope, showing a tooth implant material in powder form which is used for bone grafting in the conventional method.
FIG. 6 is a photograph of dentin collagen obtained by complete demineralization of tooth powder.
FIG. 7 is a photograph of dentin collagen obtained by complete demineralization of tooth powder, which was stained with hematoxylin and eosin and then was photographed by a microscope.
FIG. 8 is a photograph taken by an electron microscope, showing dentin collagen obtained by complete demineralization of tooth powder.
FIGS. 9 and 10 illustrate results of SDS-PAGE analysis conducted after dimerization and purification of rhBMP-2.
FIG. 11 illustrates an HPLC profile of rhBMP-2.
FIG. 12 is a graph illustrating alkaline phosphatase secretion relative to increase in rhBMP-2 dimer applied to cells.
FIG. 13 is a photograph showing a state of C2C12 cells transformed into osteoblasts upon introduction of rhBMP-2.
FIG. 14 is photographs showing a vial containing dentin collagen and a vial containing rhBMP-2.
FIG. 15 is a photograph showing a vial containing dentin collagen and rhBMP-2 in a mixed state.
FIG. 16 is a photograph showing a vial in a freeze-dried state wherein dentin collagen filled with rhBMP-2 is contained.
FIG. 17 is a photograph taken by an electron microscope, showing dentin collagen obtained by the method for manufacturing a bone graft composite according to an embodiment of the present disclosure, wherein rhBMP-2 was filled in a tissue layer of the dentin collagen, followed by freeze-drying.
FIG. 18 is photographs comparatively showing tissue states between mouse soft tissue transplanted with dentin collagen and mouse soft tissue transplanted with a composite of dentin collagen and rhBMP-2.
FIG. 19 is photographs comparatively showing tissue states between rabbit skull hard tissue transplanted with dentin collagen and rabbit skull hard tissue transplanted with a composite of dentin collagen and rhBMP-2.
FIG. 20 is photographs comparatively showing tissue states between rabbit skull hard tissues transplanted with dentin collagen and rabbit skull hard tissue transplanted with a composite of dentin collagen andrhBMP-2.
FIG. 21 is photographs comparatively showing tissue states between human bone tissue transplanted with dentin collagen and human bone tissue transplanted with a composite of dentin collagen and rhBMP-2.
FIG. 22 is a graph numerically illustrating degrees of formation of new bone, dentin and soft tissue from both of human bone tissue transplanted with dentin collagen and human bone tissue transplanted with a composite of dentin collagen and rhBMP-2.

### [DETAILED DESCRIPTION OF VARIOUS EMBODIMENTS OF THE INVENTION]

Hereinafter, embodiments of the present disclosure will be described in detail with reference to the accompanying drawings so that those skilled in the art can easily implement the present disclosure. However, the description of the present disclosure is merely an example for explanation in terms of configurations and functions, and the scope of the present disclosure should not be construed as being limited by the embodiments described in the text. That is, the embodiments are to be construed as being differently embodied and having various forms, so that the scope of the present disclosure should be understood to include equivalents capable of realizing technical ideas of the invention. Further, the purpose or effects of the present disclosure should not be construed as limiting the scope of the present disclosure, since it does not mean that a specific embodiment should include all or only such effect. Meanwhile, the meanings of terms described in the present disclosure should be understood as follows.

The terms "first", "second", and the like are intended to distinguish one element from another, and the scope to be protected should not be limited by these terms. For example, a first component may be referred to as a second component, and similarly, the second component may also be referred to as the first component.

It is to be understood that, when an element is referred to as being "connected" to another element, it may not only be directly connected to the other element, but also, other elements may be present therebetween. On the other hand, when an element is referred to as being "directly connected" to another element, it should be understood that there are no other elements therebetween. Meanwhile, other expressions describing the relationship between components, such as "between" and "immediately between" or "neighboring" and "directly adjacent to", should be interpreted as well.

It should be understood that a singular expression also includes multiple expression. Further, the terms "include" and "have" are intended to specify existence of the stated feature, number, step, operation, component, part or a combination thereof, while not precluding possibility of the presence or addition of one or more other features, numbers, steps, operations, components, parts or a combination thereof.

All terms used herein have the same meaning as commonly understood by those skilled in the art to which the present disclosure pertains, unless otherwise defined. Commonly used dictionary terms should be interpreted to be consistent with the meanings in the context of the related art, and cannot be interpreted as having ideal or excessively formal meanings unless explicitly defined in the present disclosure.

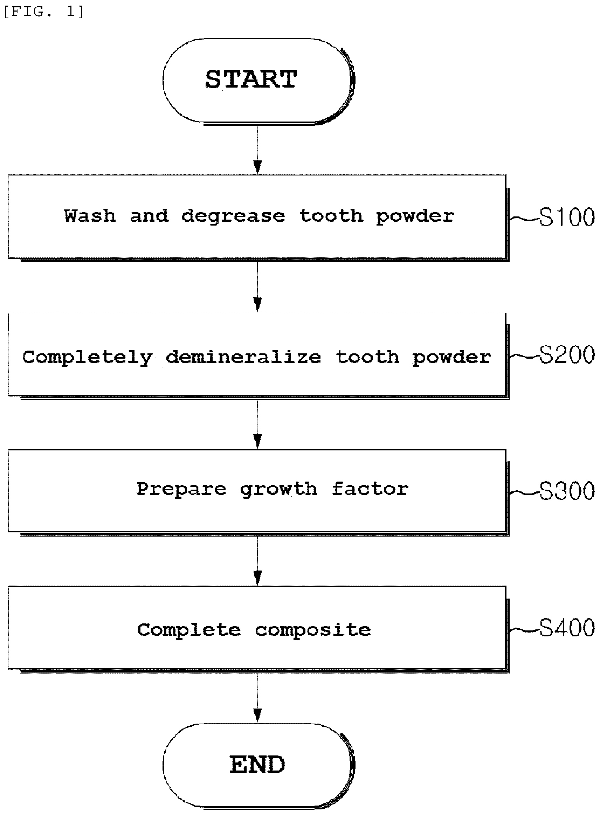

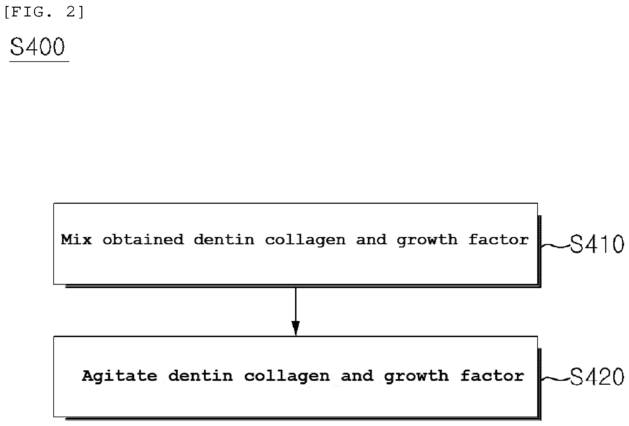

Hereinafter, a method for manufacturing a bone graft composite (hereinafter referred to as "a composite manufacturing method") according to an embodiment of the present disclosure will be described with reference to FIGS. 1 and 2.

The composite manufacturing method according to one embodiment of the present disclosure is to improve an alveolar bone regeneration rate by forming a composite of dentin collagen obtained from human teeth and a growth factor having bone inducing function. To this end, the composite manufacturing method may include washing and degreasing tooth powder (S100), completely demineralizing the tooth powder (S200), preparing a growth factor (S300) and completing a composite (S400).

Herein, the above-mentioned dentin collagen refers to a collagen mass of 100% organic material derived from a tooth that is obtained when the tooth is completely demineralized.

At first, a tooth is pulverized using a grinder to prepare tooth powder, and the tooth powder is washed and degreased using a washing machine (S100).

In one embodiment, the tooth contains collagen and bone morphogenetic protein ("BMP") consisting of the same components as alveolar bone, and thus includes substantially the same components. Further, the tooth has absorbability involved in bone reconstruction and, preferably, includes micropores formed therein, which collagen fibers of 5 to 200 µm in thickness can penetrate.

In one embodiment, the tooth used herein may be a tooth stored after extraction during dental treatment.

In one embodiment, the tooth used herein may be an autologous tooth that is the patient's own tooth, a homogenous tooth that is another person's tooth, or a heterogeneous tooth that is a tooth of an animal other than a human.

In one embodiment, in step S100 described above, the tooth may be pulverized into powder form using a grinder after removing a defect portion such as tooth decay or inflammation using a cutting tool.

In one embodiment, in step S100 described above, the tooth powder may be frozen with liquid nitrogen at -196°C or lower for 40 minutes, and then pulverized by a grinder to prepare a powder having an average particle diameter of 50 to 850um. In step S100 described above, the tooth powder is washed with distilled water for 40 minutes to remove contaminants and residual soft tissues, followed by degreasing the washed tooth powder in ethanol for 2 to 12 hours.

In one embodiment, in step S100 described above, suspended fat may be removed from the degreased tooth powder using a centrifuge, and the tooth powder may be washed in distilled water for 2 hours using a washing machine.

Next, in step S100 described above, the degreased tooth powder is completely demineralized (S200).

In one embodiment, in step S200 described above, the degreased tooth powder may be completely demineralized by replacing hydrochloric acid solution in the range of 20 times volume of 0.2 to 0.7 N for multiple times by 1 hour for 6 to 12 hours until no chemical reaction occurs.

In one embodiment, in step S200 described above, the completely demineralized tooth powder may be sterilized using a hydrogen peroxide solution for 1 hour, washed with distilled water for 2 hours, and then lyophilized. At this time, it is preferable to replace the distilled water used for washing by 30 minutes.

In step S200 described above, the completely demineralized tooth powder is transformed into dentin collagen as described above, and the dentin collagen has a collagen mass form consisting of only collagen.

In the case of tooth powder used for bone growth in the conventional method, organic and inorganic materials are obtained in a ratio by weight of 8.5:1.5 through degreasing and demineralization. On the other hand, dentin collagen obtained according to the above method includes 100% organic component.

When dentin collagen obtained by the above method is introduced into a human body, biocompatibility is excellent and more effective bone growth may be induced.

Next, a growth factor including a bone growth inducing component is prepared (S300).

In step S300 described above, the growth factor is selected from a group consisting of one of more of those, such as recombinant human bone morphogenetic protein (rhBMP-2), double complex of amino acids to construct BMP, fibroblast growth factor, growth differentiation factor (GDF), transforming growth factor (TGF), platelet-derived growth factor, insulin-like growth factor, epithelial growth factor, keratinocyte growth factor 2 (KGF2), morphogenetic protein 52(MP52), recombinant proteins thereof, and mixtures thereof.

Finally, a composite is synthesized by mixing the dentin collagen obtained from the completely demineralized tooth powder in step S200 and the growth factor prepared in step S300 described above (S400).

In one embodiment, step S400 described above may include a step of mixing the obtained dentin collagen with the growth factor S410, and a step of stirring the obtained growth factor as well as the obtained dentin collagen S420.

In step 410 described above, the pH of the dentin collagen and the growth factor in a mixed state is maintained in a range of pH 6.5 to 8.

In step S420 described above, the mixture of the dentin collagen and growth factor is stirred using a shaker at 30 to 80 rpm for 3 to 5 minutes.

Unlike the conventional method of coating the surface of tooth powder (=tooth-derived collagen and inorganic material) with the growth factor via the above-described step S420, a layer filling method of filling a tissue layer of dentin collagen with the growth factor rhBMP-2 may be adopted so as to improve an ability to transfer the growth factor to a defect site.

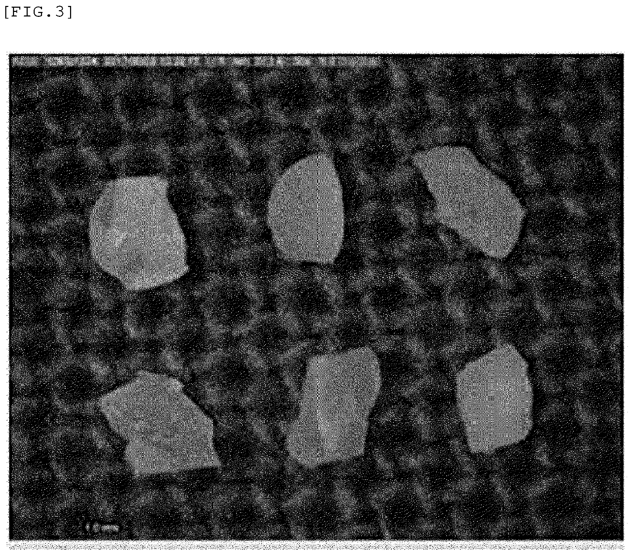

Hereinafter, an embodiment of the method for manufacturing the composite described above will be described with reference to FIGS. 3 to 22.

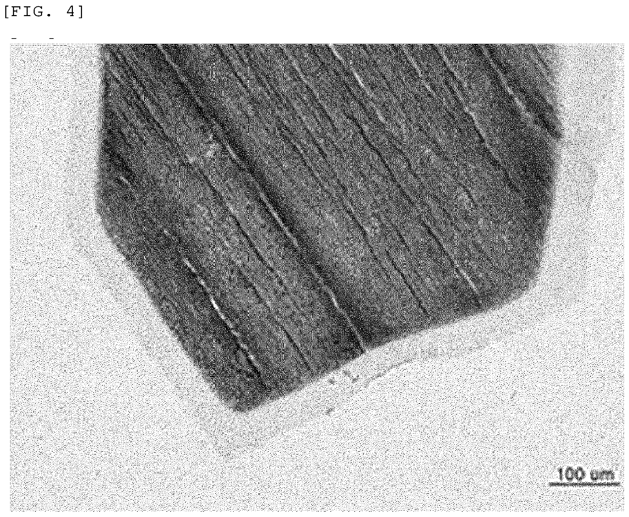

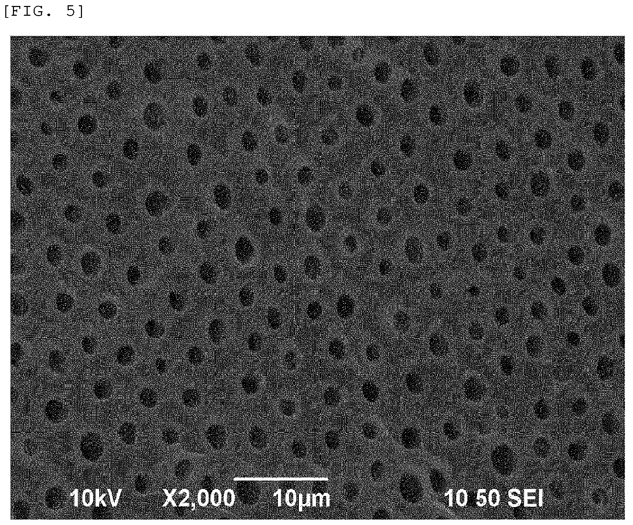

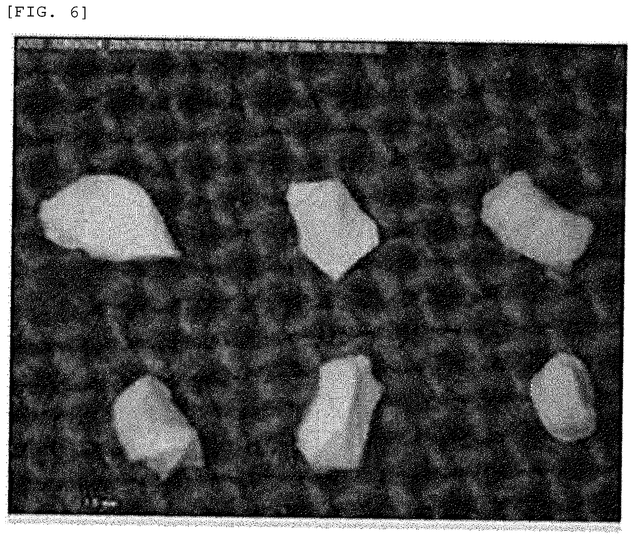

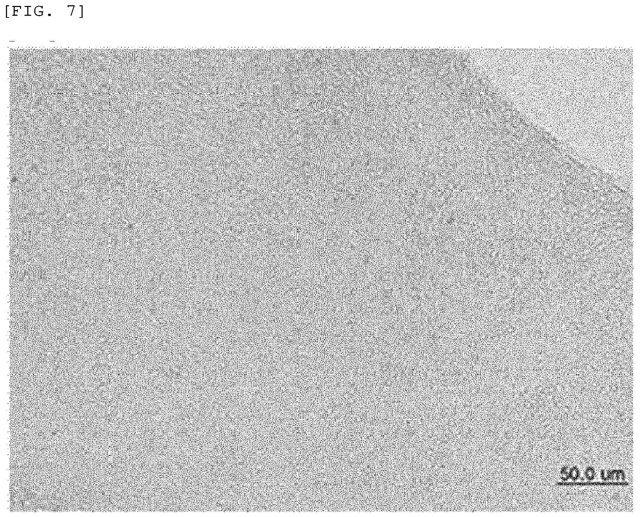

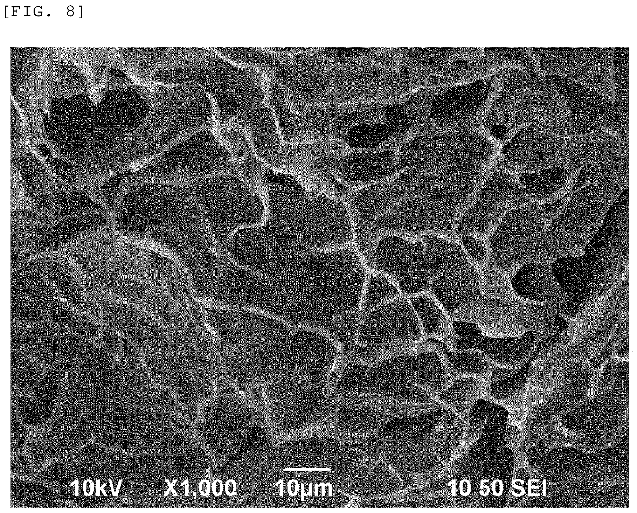

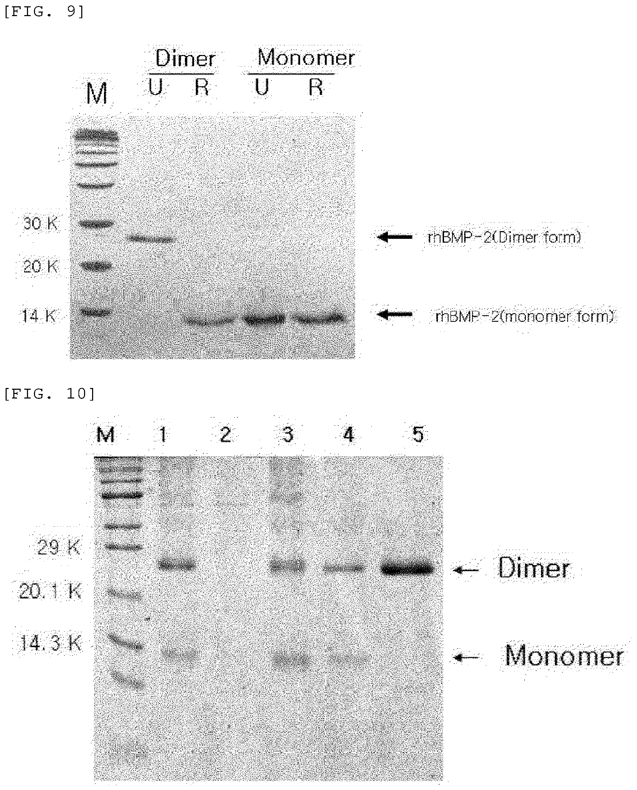

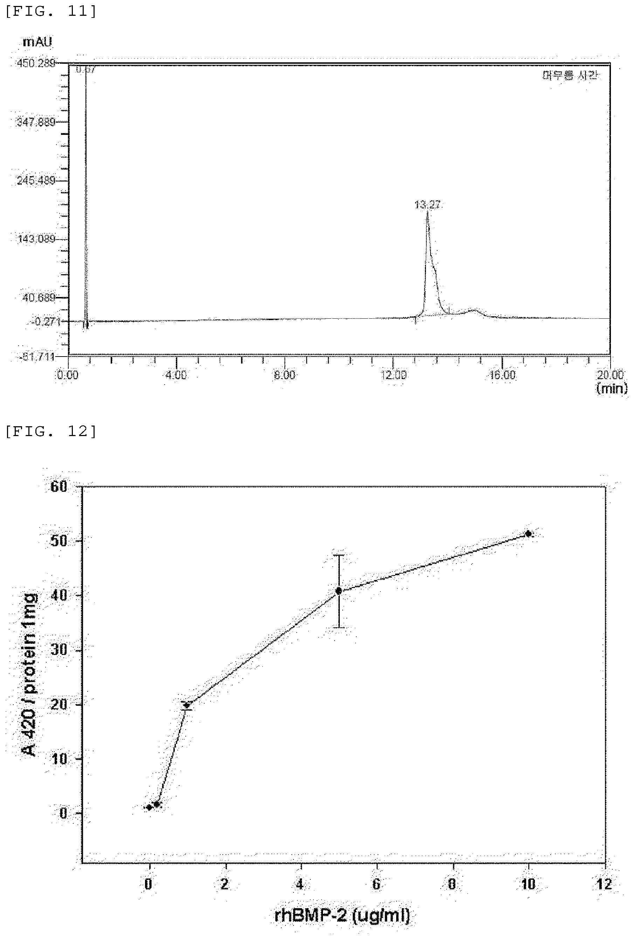

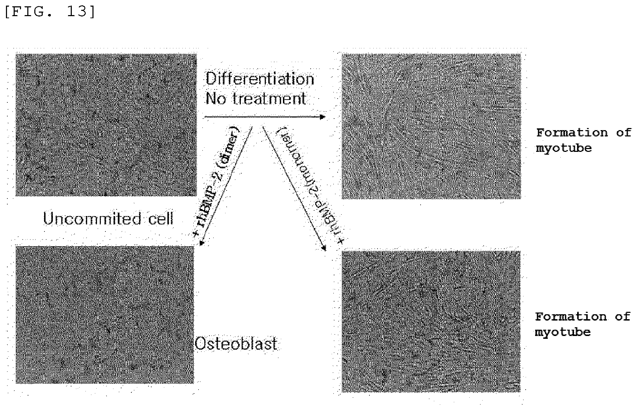

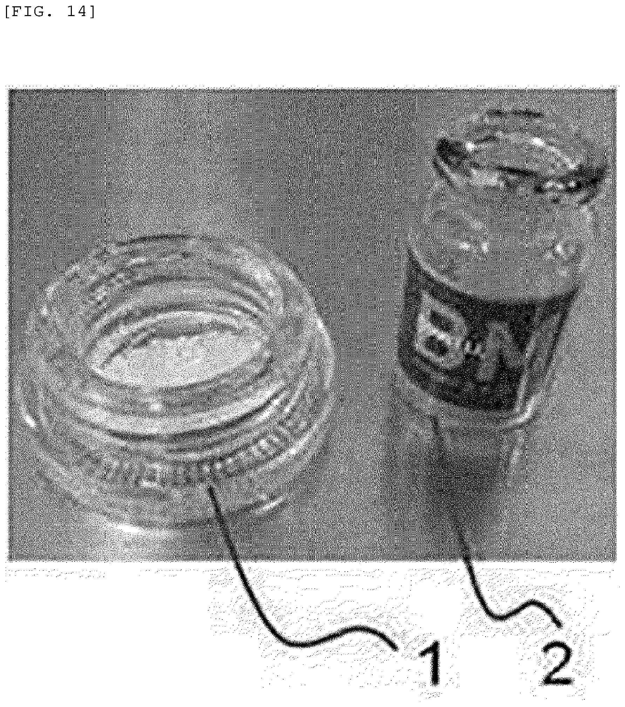

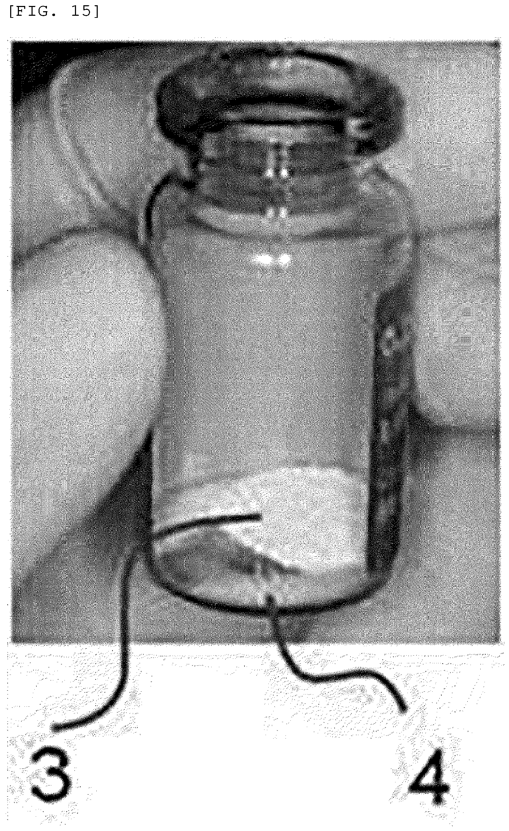

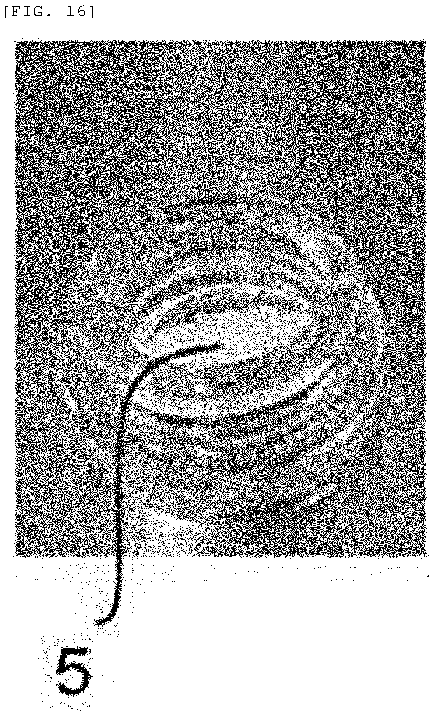

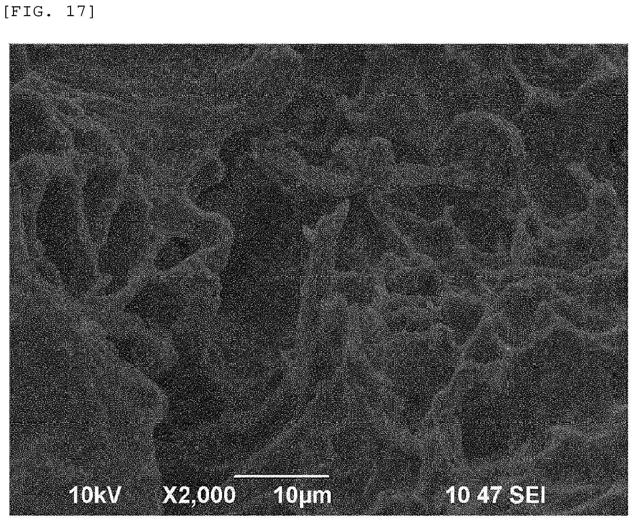

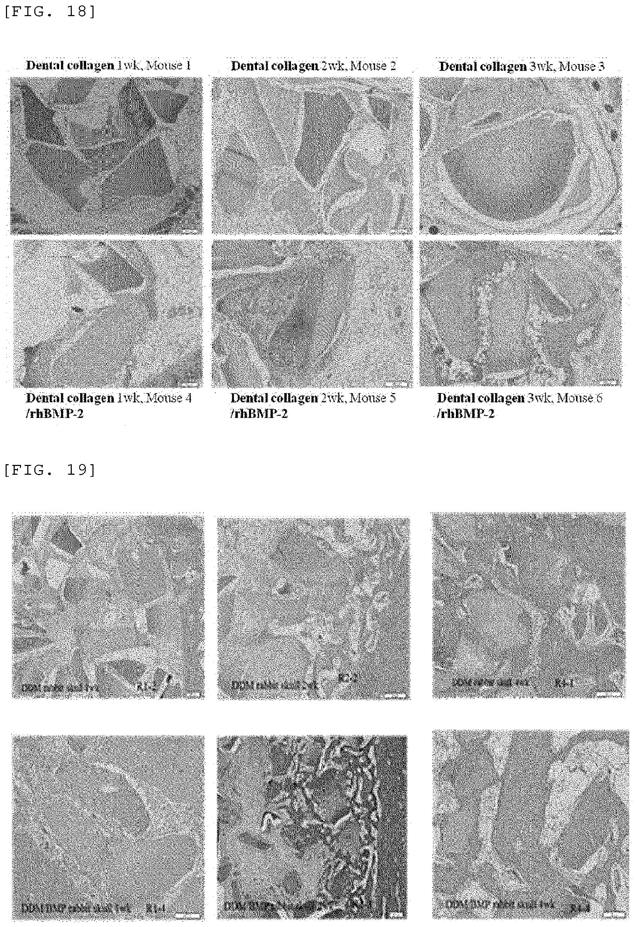

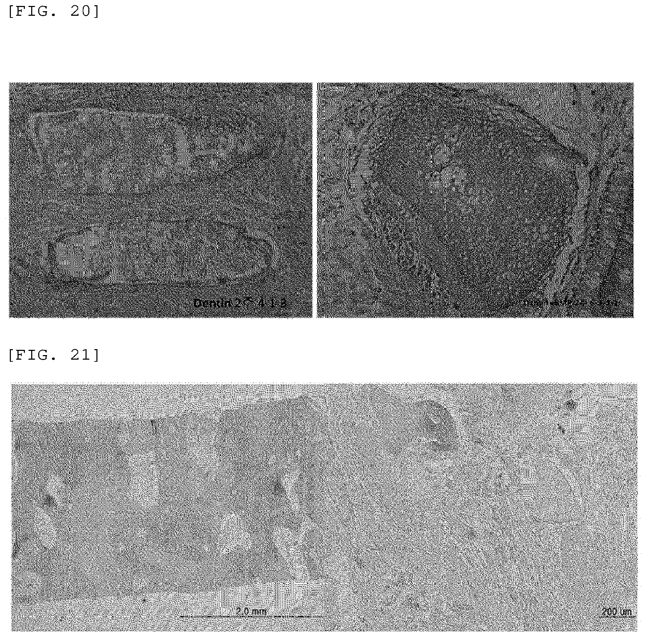

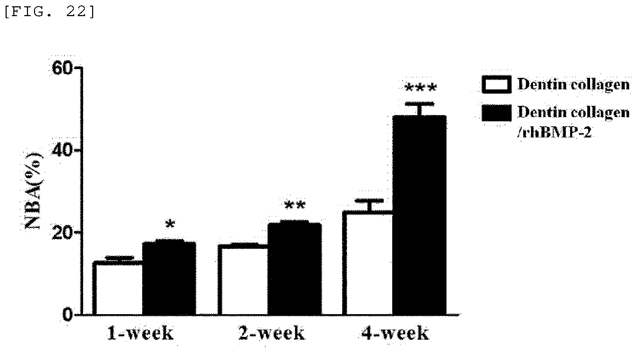

FIG. 3 is a photograph of a tooth implant material in powder form which is used for bone grafting in the conventional method; FIG. 4 is a photograph of the tooth implant material in powder form used for bone grafting in the conventional method , wherein the tooth implant material was stained with hematoxylin and eosin and then was photographed by a microscope; FIG. 5 is a photograph taken by an electron microscope, showing a tooth implant material in powder form which is used for bone grafting in the conventional method; FIG. 6 is a photograph of dentin collagen obtained by complete demineralization of tooth powder; FIG. 7 is a photograph of dentin collagen obtained by complete demineralization of tooth powder, which was stained with hematoxylin and eosin and then was photographed by a microscope; FIG. 8 is a photograph taken by an electron microscope, showing dentin collagen obtained by complete demineralization of tooth powder; FIGS. 9 and 10 illustrate results of SDS-PAGE analysis conducted after dimerization and purification of rhBMP-2; FIG. 11 illustrates an HPLC profile of rhBMP-2; FIG. 12 is a graph illustrating alkaline phosphatase secretion relative to increase in rhBMP-2 dimer applied to cells; FIG. 13 is a photograph showing a state of C2C12 cells transformed into osteoblasts upon introduction of rhBMP-2; FIG. 14 is photographs showing a vial containing dentin collagen and a vial containing rhBMP-2; FIG. 15 is a photograph showing a vial containing dentin collagen and rhBMP-2 in a mixed state; FIG. 16 is a photograph showing a vial in a freeze-dried state wherein dentin collagen filled with rhBMP-2 is contained; FIG. 17 is a photograph taken by an electron microscope, showing dentin collagen obtained by the method for manufacturing a bone graft composite according to an embodiment of the present disclosure, wherein rhBMP-2 was filled in a tissue layer of the dentin collagen, followed by freeze-drying. Further, FIG. 18 is photographs comparatively showing tissue states between mouse soft tissue transplanted with dentin collagen and mouse soft tissue transplanted with a composite of dentin collagen and rhBMP-2; FIG. 19 is photographs comparatively showing tissue states between rabbit skull hard tissue transplanted with dentin collagen and rabbit skull hard tissue transplanted with a composite of dentin collagen and rhBMP-2; FIG. 20 is photographs comparatively showing tissue states between rabbit skull hard tissue transplanted with dentin collagen and rabbit skull hard tissue transplanted with a composite of dentin collagen and rhBMP-2; FIG. 21 is photographs comparatively showing tissue states between human bone tissue transplanted with dentin collagen and human bone tissue transplanted with a composite of dentin collagen and rhBMP-2; and FIG. 22 is a graph numerically illustrating degrees of formation of new bone, dentin and soft tissue through both of human bone tissue transplanted with dentin collagen and human bone tissue transplanted with a composite of dentin collagen and rhBMP-2.

### [EXAMPLE]

### Example 1: Preparation of autologous teeth

A tooth was prepared from the patient himself (wisdom tooth, healthy tooth such as premolars extracted for correction purposes), and defects such as tooth decay and inflammation were removed.

The tooth in powder form was frozen using liquid nitrogen at -196°C or lower for 40 minutes and then pulverized by a grinder to form a powder having an average particle size of 50 to 850 µm as shown in FIG. 3. The powder was washed with distilled water for 40 minutes to remove contaminants and residual soft tissue, and the washed powder was degreased with ethanol for 2 to 12 hours. The degreased powder was centrifuged to remove suspended fat and then washed with distilled water for 2 hours. The aqueous solution was completely demineralized by replacing a hydrochloric acid solution in the range of 20 times volume of 0.2 to 0.7 N by 1 hour for 6 to 12 hours until no chemical reaction occurred. The completely demineralized powder was washed with distilled water for 2 hours. Following this, the powder was disinfected with hydrogen peroxide solution for 1 hour and washed with distilled water for 2 hours (while replacing distilled water every 30 minutes), followed by freeze-drying. By this complete demineralization, only the collagen component (organic material) remained.

### Example 2: Preparation of rhBMP-2

### 1. Production of rhBMP-2

### (1) Expression and purification of human BMP-2 gene using E. coli

In order to obtain the human BMP-2 gene, total cellular RNA was extracted from U2OS cells (Life technologies TM, Korea) using Trizol solution (Gibco BRL, USA) in U2OS cells and was subjected to reverse transcription. Polymerase chain reaction (PCR) was performed using 5'-AGAAGAACATATGCAAGCCAAACACAAACAGCGG-3 as a sense primer and 5'-AATTTTACAGCTTCTAGCGACACCCACAACCCT-3' as an antisense primer with cDNA as a template. The PCR product was isolated and inserted into pGEM-T vector (Promega, USA) and cloned using E. coli (DH5α) (Life technologies^{™}, Korea).

### (2) High-density cell culture

High-density culture was performed in a fermenter (KoBioTec, Korea) using Fatemech et al. technique (Tabandeh F, Shojaosadati SA, Zomorodipour A, et al. Heat-induced production of human growth hormone by high density cultivation of recombinant Escherichia coli. Biotechnol Lett 2004; 26: 245-250. Korz DJ, Rinas U, Hellmuth K, Sanders EA, Deckwer W-D. Simple fed-batch technique for high cell density cultivation of Escherichia coli. J of Biotechnology 1995; 39:59-65). A sterile nutrient medium (glucose 33.3 g/L, peptone 10 g/L, yeast extract 5 g/L, MgSO₄ 1 g/L, CaCl₂ 0.048 g/L, ZnSO₄ 0.0176 g/L, CuSO₄ 0.008 g/L) was added thereto and cultured for 24 hours with stirring at a stirring speed of 250 rpm.

### (3) Protein purification

The suspension was stored in a cryogenic freezer at -80°C (Nihon Freezer, Japan). The frozen suspension was thawed at a refrigeration temperature, and the cells were disrupted by pressurization and then centrifuged at 5,500 g and 4°C for 45 minutes. When the mature rhBMP-2 having undergone a re-denaturalization process has the original tertiary structure, N-terminus has a heparin-binding site.

### 2. Biochemical properties of purified rhBMP-2

(1) Purity and identification test of rhBMP-2 stock solution As a result of the SDS-PAGE test, as shown in FIG. 10, the rhBMP-2 stock solution showed the same migration distance as the standard solution and exhibited a purity of 95% or more.
(2) HPLC (High performance Liquid Chromatography) analysis The purified rhBMP-2 dimer was dissolved in 0.1% TFA (trifluoroacetic acid) at a concentration of 1 µg/µl and subjected to detection and monitoring of protein in each fraction using a C4 reversed-phase HPLC column (4.6 mm X 50 mm, 300 Å, 5 µm particle size; GraceVydac, United States). As shown in FIG. 11, the test results of the rhBMP-2 stock solution showed that a retention time is substantially equal to that of the standard solution, confirming that it was purified into pure rhBMP-2.

### 3. Production and purification results of rhBMP-2 protein

After dimerization, affinity chromatography was performed in a heparin column (Sigma Dow, Italy). As a result, as shown in FIG. 10, most monomers and a small amount of dimer were eluted from 0.3 M NaCl fraction, while the dimers were eluted from 0.5 M NaCl fraction. Purified rhBMP-2 monomers and dimers were identified by SDS-PAGE analysis. The size of the produced monomer was calculated to be about 114 amino acid residues and the molecular weight of the monomer was about 14 kDa. The dimer has the same size of band as the monomer in non-reductive conditions since two monomers are connected by disulfide bonds. The size of the dimer was about 28 kDa.

### 4. Biological activity of purified rhBMP-2 (in vitro test)

Referring to FIGS. 12 and 13, rhBMP-2 dimer was applied to C2C12 cells (Sigma Dow, Italy), which are myoblasts. As a result, 3 days after culture, it could be seen that alkaline phosphatase as a representative protein of osteoblasts is secreted and the cells are transformed into osteoblasts. Thus, it was confirmed that rhBMP-2 functions as a bone morphogenetic protein.

### Example 3: Preparation of bone graft composite containing growth factor

A bone graft composite according to the present disclosure in which the rhBMP-2 in Example 2 was filled in a tissue layer of dentin collagen derived from the tooth in Example 1 was prepared.

Hereinafter, for example, the bone graft composite according to the present disclosure, in which the rhBMP-2 in Example 2 was filled into the tissue layer of dentin collagen derived from the tooth in Example 1 will be described.

1 g of completely demineralized dentin collagen was dispensed into a glass vial and capped with a silicone plug. 1 mg of rhBMP-2 was dissolved in a solution consisting of 5 mM glutamic acid, 2.5% by weight ("wt.%") glycine, 5 mM sodium chloride, 0.015 wt.% of Tween-80 and 0.5 wt.% of D-sorbitol, and the solution was added to the glass vial in which the tooth powder was immersed. The completely demineralized tooth implant material and the vial containing rhBMP-2 were stirred at 4 to 20°C using a shaker at 30 to 80 rpm for 3 to 5 minutes. The pH was maintained at 6.5 to 8, while rhBMP-2 penetrated into the inner layer of the tooth implant material by adding a physical element using a shaker. Thereafter, the glass vial was placed and maintained in a vacuum freeze dryer at -80°C for 3 to 6 hours, followed by gradually elevating the temperature to 20°C to minimize damage to rhBMP-2. The freeze-dried glass vial was sterilized with ethylene oxide.

### Experimental Example: Test Example of BMP

Six athymic mice (average weight: 15 to 20 g) were used. The animals were divided into three groups (1, 2 and 4 weeks), and each group was divided into two subgroups: a tooth administration group and a growth factor administration group. The tooth administration group is an experimental group in which only dentin collagen was administered, and the growth factor administration group is a control group in which a composite of dentin collagen and growth factor rhBMP-2 was administered. Six athymic mice were fed with free access to food and water under a mean ambient temperature condition of 22°C and at a 12-hour shading cycle. Pentobarbital sodium (tranquilizer) was administered intraperitoneally and an operation site was disinfected and isolated to perform general anesthesia. A thigh portion was incised to form subcutaneous pockets on both sides and grafts of the growth factor administration group (0.03 g of dentin collagen, 0.2 mg/ml, 5.0 µg of rhBMP-2) were used, wherein the control group was applied to the left side while the experimental group was applied to the right side.

In the growth factor administration group, 0.03 mg of tissue layer of dentin collagen in the bone graft composite in Example 3 was filled with 0.2 ml of aqueous solution of rhBMP-2. In the control group, only 0.03 mg of dentin collagen without the growth factor was inserted and then the incision was sutured with a nylon suture. In order to protect the wound from infection, antibiotic ointment (Daewoong Pharm, Korea) was used throughout the study.

Soft tissue biopsies containing dentin collagen and the growth factor rhBMP-2 in the subcutaneous pocket were obtained from the athymic mice at 1, 2 and 4 weeks after insertion. A total of 12 biopsies was fixed in 10% formalin for 24 hours. After complete demineralization in Calci-Clear Rapid (National Diagnostics, Atlanta, GA) for 12 hours, the specimens were washed with distilled water. The specimens were then treated by a Hypercentre XP tissue processor (Shandon, UK), submerged in paraffin, and cut to 4 to 5 µm thickness. The divided specimens were observed using an optical microscope after hematoxylin and eosin staining. Images were captured using a Magna Fire digital camera system (Optronics, Goleta, Calif.).

FIG. 18 is photographs of specimens after 1 week, 2 weeks and 4 weeks, wherein soft tissue generation was most active in the growth factor administration group while being slowest in the tooth administration group.

However, it was found that the actual alveolar bone in a magnified image of the specimen after 1 week is produced in a completely different manner. That is, in the growth factor administration group, the regenerated bone is larger than the original tooth particle, whereas the tooth administration group showed a part of the alveolar bone to be regenerated on only the left side.

Referring to the photograph of the specimens after 2 weeks, it could be seen that the growth factor administration group (4 weeks later) achieved regeneration. On the other hand, a part of the tooth in Example 1 which is the tooth administration group (4 weeks later) was still observed, indicating that an alveolar bone regeneration rate is lower than that of the bone graft composite according to the present disclosure.

Finally, in the photographs of the specimens after 4 weeks, cartilage formation was demonstrated, which is evidence of bone induction. More particularly, similar to the growth factor administration group, the tooth administration group at the left side also exhibited cartilage. Further, tooth particles were never observed in the growth factor administration group. These results indicate that the cartilage was completely restored in addition to alveolar bone regeneration.

Referring to FIG. 19, 12 male rabbits (2.50 to 3.00 kg body weight) were under standard experimental pellet feed through free experiments at ambient temperature of 9 to 21°C. Rabbits were anesthetized by intramuscular injection (5 mg/kg body weight) of a 4:1 solution of ketamine hydrochloride and xylene. The surgical site was shaved and rubbed with iodine. In the case of a skull defect model, sagittal incision was made across the skull, and the head and neck bone were exposed while reflecting full thickness flap. 4 circular defects with 8 mm diameter were created in the skull of each rabbit using a trephine drill. Each group received the following treatment: tooth powder administration control groups (0.03 g) in two defects (n = 12) on the left side; and growth factor administration groups (0.03 g of dentin collagen, 0.2 mg/ml, 5.0 g of rhBMP-2) in two other defects (n = 12) on the right side. All surgical sites were primarily sutured with sutures (4-0 Monosyn). Rabbits were sacrificed at 1, 2 and 4 weeks post-transplantation for radiological and histological assessment. As a result of FIG. 19, both of the tooth administration group and the growth factor administration group did not show obvious difference at 1 week except that the bone was formed with a configuration of defect margin. At 2 weeks, new bone formation was observed in the tooth powder sample and such newly formed bone at the margin was prominent in the growth factor administration group. The newly formed bone at 4 weeks was continuously grown from the defect margin of the tooth powder administration group to the center thereof, resulting in high-density fibrous connective tissue. The growth factor administration group exhibited significant increase in new bone formation.

Dentin collagen in FIG. 20 showed collagen degradation by protease and surface bone inducing ability, while the growth factor administration group showed bone inducing ability, as well as expansion of inner dentinal tubules and activation of bone-inducing cartilage cells in the cartilage, thereby demonstrating sufficient activity of rhBMP-2.

In FIGS. 21 and 22, 10 implant patients were selected. 5 portions (one in the maxilla and four in the mandible) were restored as control sites by administration of dentin collagen alone, while the other 5 portions (three in the maxilla and two in the mandible) were restored as an experimental sites by the growth factor administration group. Criteria to satisfy the operation were: (1) patients requiring implant placement and alveolar bone reinforcement; (2) patients older than 20 years of age; (3) patients with chronic peri-implantitis or aseptic condition as causes of implant removal; and (4) patients with good health or well controlled systemic disease. Patients who met one of the following exclusion criteria were not included in the study: (1) patients who received graft removal due to fixture fracture and laceration of internal granulation tissue according to mechanical causes: (2) patients with severe systemic disease such as recent heart attacks or coagulation disorders; (3) smokers; (4) patients who received chemotherapy or radiotherapy; and (5) patients with acute infection, chronic inflammation such as implant induced chronic sinusitis.

The collected tooth was stored in 75% alcohol, stored in a refrigerator or freezer and sent for treatment to a clean room of Korea Tooth Bank Co. Ltd. The soft tissues remaining in the clean room were removed, and the tooth was crushed into particles of 50 to 850 µm in diameter, washed, degreased, desalted and lyophilized as previously reported. After sterilization with ethylene oxide, tooth powder was stored at room temperature for clinical use. The growth factor administration group was prepared by adding 5.0 µg of rhBMP-2 (Cowellmedi, Busan, Korea) to 0.03 g of tooth powder (0.2 mg/ml rhBMP-2 concentration). The mixture was deeply frozen at -70°C and placed in a freeze-dried glass vial, followed by fixing the same in a freeze dryer (ILShin Lab, Seoul, Korea).

Surgery was performed under local anesthesia with 2% lidocaine HCl containing epinephrine. After incision and flap opening, tooth powder or a composite synthesized from dentin collagen and growth factor rhBMP-2 was used for supplement. Patients were instructed to take oral antibiotics (625 mg, amoxicillin and clavulanate, Augmentin, Ilseong Pharmaceutical, Seoul) 3 times a day for 5 days after rinsing the mouth twice a day with 0.1% chlorhexidine solution. At the 7th day of observation, calm healing was observed in all areas. Patients with signs of inflammation, infection or graft rejection were not reported.

The graft site was re-incised at the end of 3 months for the mandible and at 6 months for the maxillary in order to collect tissues (Trephine drill: 3.0 mm outer diameter and 2.3 mm inner diameter, Dentium, Seoul, Korea). Each sample was prepared to include both newly formed bone and implanted dentin particles. There were no wound complications such as hematoma, wound dehiscence, postoperative fever, and hemorrhage.

The tissue harvested by the trephine drill was processed for histological assessment. Two sections containing entire defect were selected and stained with hematoxylin and eosin. Digital images were obtained, and (1) a ratio of newly formed bone site, (2) an area ratio of dentin base material residue, and (3) a ratio of soft tissue component area proportional to the total area were determined using test tubes with unknown types of graft materials.

Referring to FIG. 21, the histological assessment demonstrated that tooth powder exhibits bone induction and osteo-conductive bone formation around tooth powder particles. The tooth powder particles were surrounded by dense fibrous connective tissues. On the other hand, the growth factor administration group showed active bone formation in osteogenesis and osteoblast inherence. In the growth factor administration group, fibroblast infiltration as well as collagen degradation and absorption of dentin matrix, which is associated with tooth powder of new bone re-absorbed in multinuclear giant cells, were observed.

Referring to FIG. 22, the ratio of the newly formed bone was 34.39 ± 27.70 in the growth factor administration group and 27.62 ± 12.84 in the tooth administration group, respectively. The mean percentage of residual dentin was 5.82 ± 5.10 in the growth factor administration group and 18.54 ± 18.06 in the tooth administration group, respectively. The mean percentage of soft tissue components was 44.29 ± 26.26 in the growth factor administration group and 31.98 ± 15.88 in the tooth administration group, respectively. The amounts of new bone formation and soft tissues were very similar between both groups with no statistically significant difference.

Such a bone graft composite as described above may induce rapid regeneration of alveolar bone to thus greatly reduce patient discomfort and achieve additional effects such as prevention of infection caused by long-term regeneration, thereby being very useful in the field of medical care.

## Claims

1. A method for manufacturing composite for bone grafting, comprising:
a. pulverizing a tooth to generate tooth powder, and washing and degreasing the tooth powder;
b. completely demineralizing the tooth powder to obtain dentin collagen as a collagen mass of 100% organic material derived from a tooth that is obtained when the tooth powder is completely demineralized;
c. preparing a growth factor containing a bone growth-inducing component; and
d. synthesizing a composite by mixing the obtained dentin collagen with the growth factor,
wherein in the step for washing and degreasing of the tooth powder, the tooth powder is washed with distilled water for 40 minutes to remove contaminants and residual soft tissues, followed by degreasing the washed tooth powder in ethanol for 2 to 12 hour,
wherein the step for completely demineralizing the tooth powder includes completely demineralizing degreased tooth powder in a hydrochloric acid solution in the range of 20 times volume of 0.2 to 0.7 N for 6 to 12 hours,
wherein the step for synthesizing the composite includes:
a. mixing the obtained dentin collagen and growth factor; and
b. agitating the mixture of the obtained dentin collagen and growth factor at 30 to 80 rpm for 3 to 5 minutes, and the growth factor in a mixed state are maintained at pH 6.5 to 8, and
wherein the growth factor is selected from a group consisting of one or more of those, such as recombinant human bone morphogenetic protein (rhBMP-2), double complex of amino acids to construct BMP, fibroblast growth factor, growth differentiation factor(GDF), transforming growth factor (TGF), platelet-derived growth factor, insulin-like growth factor, epithelial growth factor, keratinocyte growth factor 2 (KGF2), morphogenetic protein 52 (MP52), recombinant proteins thereof, and mixtures thereof.

2. A bone graft composite manufactured by the method of claim 1.

## Patentansprüche

1. Ein Verfahren zum Herstellen eines Komposits zur Knochentransplantation, das folgende Schritte aufweist:
a. Pulverisieren eines Zahns, um Zahnpulver zu erzeugen, und Waschen und Entfetten des Zahnpulvers;
b. vollständiges Entmineralisieren des Zahnpulvers, um Dentinkollagen als eine Kollagenmasse von 100% organischem Material zu erhalten, das von einem Zahn stammt, welches erhalten wird, wenn das Zahnpulver vollständig entmineralisiert ist;
c. Herstellen eines Wachstumsfaktors, der eine knochenwachstumsinduzierende Komponente enthält; und
d. Synthetisieren eines Komposits durch Mischen des erhaltenen Dentinkollagens mit dem Wachstumsfaktor,
wobei bei dem Schritt des Waschens und Entfettens des Zahnpulvers das Zahnpulver für 40 Minuten mit destilliertem Wasser gewaschen wird, um Verunreinigungen und Restweichgewebe zu entfernen, gefolgt von Entfetten des gewaschenen Zahnpulvers in Ethanol für 2 bis 12 Stunden,
wobei der Schritt des vollständigen Entmineralisierens des Zahnpulvers ein vollständiges Entmineralisieren von entfettetem Zahnpulver in einer Salzsäurelösung im Bereich des 20-fachen Volumens von 0,2 bis 0,7 N für 6 bis 12 Stunden umfasst,
wobei der Schritt des Synthetisierens des Komposits folgende Schritte umfasst:
a. Mischen des erhaltenen Dentinkollagens und des Wachstumsfaktors; und
b. Rühren der Mischung des erhaltenen Dentinkollagens und des Wachstumsfaktors bei 30 bis 80 U/min für 3 bis 5 Minuten, wobei der Wachstumsfaktor in einem gemischten Zustand bei pH 6,5 bis 8 gehalten wird, und
wobei der Wachstumsfaktor aus einer Gruppe ausgewählt ist, die aus einem oder mehreren der folgenden besteht, beispielsweise rekombinantem humanem knochenmorphogenetischem Protein (rhBMP-2), Doppelkomplex von Aminosäuren zum Konstruieren von BMP, Fibroblastenwachstumsfaktor, Wachstumsdifferenzierungsfaktor (GDF), transformierendem Wachstumsfaktor (TGF), von Blutplättchen abgeleitetem Wachstumsfaktor, insulinähnlichem Wachstumsfaktor, Epithelwachstumsfaktor, Keratinozytenwachstumsfaktor 2 (KGF2), morphogenetischem Protein 52 (MP52), rekombinanten Proteinen davon und Mischungen davon.

2. Ein Knochentransplantat-Komposit, das durch das Verfahren gemäß Anspruch 1 hergestellt wird.

## Revendications

1. Procédé de fabrication de composite pour greffe osseuse, comprenant :
a. la pulvérisation d'une dent pour générer de la poudre dentaire, et le lavage et le dégraissage de la poudre dentaire ;
b. la déminéralisation complète de la poudre dentaire pour obtenir du collagène de dentine sous forme de masse de collagène de matière 100 % organique dérivée d'une dent qui est obtenue lorsque la poudre dentaire est complètement déminéralisée ;
c. la préparation d'un facteur de croissance contenant un composant inducteur de croissance osseuse ; et
d. la synthétisation d'un composite par mélange du collagène de dentine obtenu avec le facteur de croissance,
dans lequel, dans l'étape de lavage et de dégraissage de la poudre dentaire, la poudre dentaire est lavée avec de l'eau distillée pendant 40 minutes pour éliminer les contaminants et les tissus mous résiduels, puis la poudre dentaire lavée est dégraissée dans de l'éthanol pendant 2 à 12 heures,
dans lequel l'étape de déminéralisation complète de la poudre dentaire inclut la déminéralisation complète de la poudre dentaire dégraissée dans une solution d'acide chlorhydrique dans la plage de 20 fois le volume de 0,2 à 0,7 N pendant 6 à 12 heures,
dans lequel l'étape de synthèse du composite inclut :
a. le mélange du collagène de dentine obtenu et du facteur de croissance ; et
b. l'agitation du mélange du collagène de dentine obtenu et du facteur de croissance à 30 à 80 tr/min pendant 3 à 5 minutes, et le facteur de croissance à un état mélangé sont maintenus à un pH de 6,5 à 8, et
dans lequel le facteur de croissance est sélectionné dans un groupe consistant en un ou plusieurs de ceux-ci, tels que la protéine morphogénétique osseuse humaine recombinante (rhBMP-2), le double complexe d'acides aminés pour construire la BMP, le facteur de croissance des fibroblastes, le facteur de différenciation de la croissance (GDF), le facteur de croissance transformant (TGF), le facteur de croissance dérivé des plaquettes, le facteur de croissance insulinomimétique, le facteur de croissance épithéliale, le facteur de croissance des kératinocytes 2 (KGF2), la protéine morphogénétique 52 (MP52), leurs protéines recombinantes, et leurs mélanges.

2. Composé de greffe osseuse fabriqué selon le procédé de la revendication 1.
